# EUROPEAN PATENT APPLICATION

(11) **EP 3 896 089 A1**
(43) Date of publication of application: **20.10.2021**
(21) Application number: 19897174.9
(22) Date of filing: 12.12.2019
(51) Int. Cl.: C07K 19/00, C12N 15/62, C12N 15/85, A61K 38/20, A61K 39/395, A61P 35/00, A61P 7/00

(54) **USE OF IL-15 PROTEIN COMPLEX JOINT PD-L1 ANTIBODY FOR TREATING TUMOR DISEASES**

(30) Priority: 13.12.2018 CN 201811526262; 11.01.2019 CN 201910026017
(71) Applicant: Jiangsu Hengrui Medicine Co., Ltd., Lianyungang, Jiangsu 222047 (CN); Suzhou Suncadia Biopharmaceuticals Co., Ltd., Suzhou, Jiangsu 215126 (CN)
(72) Inventor: SUN, Xing, Lianyungang, Jiangsu 222047 (CN); MA, Chen, Lianyungang, Jiangsu 222047 (CN); YANG, Changyong, Lianyungang, Jiangsu 222047 (CN); LIAO, Cheng, Lianyungang, Jiangsu 222047 (CN); ZHANG, Lianshan, Lianyungang, Jiangsu 222047 (CN); ZOU, Jianjun, Lianyungang, Jiangsu 222047 (CN); SHI, Wei, Lianyungang, Jiangsu 222047 (CN); DU, Yuanyuan, Lianyungang, Jiangsu 222047 (CN); ZHOU, Yujie, Lianyungang, Jiangsu 222047 (CN)
(74) Representative: Potter Clarkson
(86) International application number: PCT/CN2019/124833
(87) International publication number: WO 2020/119758

(57) **Abstract**

Provided is use of IL-15 protein complex joint PD-L1 antibody for treating tumor diseases.

Sequence listing

<110>  JIANGSU HENGRUI MEDICINE CO., LTD.,
       SUZHOU SUNCADIA BIOPHARMACEUTICALS CO., LTD.

<120>  USE OF IL-15 PROTEIN COMPLEX JOINT PD-L1 ANTIBODY FOR TREATING TUMOR
DISEASES

<160>  18

<170>  SIPOSequenceListing 1.0

<210>  1
<211>  5
<212>  PRT
<213>  Artificial Sequence

<400>  1
Ser Tyr Trp Met His
1               5

<210>  2
<211>  17
<212>  PRT
<213>  Artificial Sequence

<400>  2
Arg Ile Xaa Pro Asn Ser Gly Xaa Thr Ser Tyr Asn Glu Lys Phe Lys
1               5                   10                  15
Asn

<210>  3
<211>  10
<212>  PRT
<213>  Artificial Sequence

<400>  3
Gly Gly Ser Ser Tyr Asp Tyr Phe Asp Tyr
1               5                   10

<210>  4
<211>  15
<212>  PRT
<213>  Artificial Sequence

<400>  4
Arg Ala Ser Glu Ser Val Ser Ile His Gly Thr His Leu Met His
1               5                   10                  15

<210>  5
<211>  7
<212>  PRT
<213>  Artificial Sequence

<400>  5
Ala Ala Ser Asn Leu Glu Ser
1               5

<210>  6
<211>  9
<212>  PRT
<213>  Artificial Sequence

<400>  6
Gln Gln Ser Phe Glu Asp Pro Leu Thr
1               5

<210>  7
<211>  119
<212>  PRT
<213>  Artificial Sequence

<400>  7
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Gly Leu Glu Trp Met
        35                  40                  45
Gly Arg Ile Gly Pro Asn Ser Gly Phe Thr Ser Tyr Asn Glu Lys Phe
    50                  55                  60
Lys Asn Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Gly Ser Ser Tyr Asp Tyr Phe Asp Tyr Trp Gly Gln Gly
            100                 105                 110
Thr Thr Val Thr Val Ser Ser
        115

<210>  8
<211>  111
<212>  PRT
<213>  Artificial Sequence

<400>  8
Asp Ile Val Leu Thr Gln Ser Pro Ala Ser Leu Ala Val Ser Pro Gly
1               5                   10                  15
Gln Arg Ala Thr Ile Thr Cys Arg Ala Ser Glu Ser Val Ser Ile His

                20                  25                  30
Gly Thr His Leu Met His Trp Tyr Gln Gln Lys Pro Gly Gln Pro Pro
        35                  40                  45
Lys Leu Leu Ile Tyr Ala Ala Ser Asn Leu Glu Ser Gly Val Pro Ala
    50                  55                  60
Arg Phe Ser Gly Ser Gly Ser Gly Thr Asp Phe Thr Leu Thr Ile Asn
65                  70                  75                  80
Pro Val Glu Ala Glu Asp Thr Ala Asn Tyr Tyr Cys Gln Gln Ser Phe
                85                  90                  95
Glu Asp Pro Leu Thr Phe Gly Gln Gly Thr Lys Leu Glu Ile Lys
            100                 105                 110

<210>  9
<211>  446
<212>  PRT
<213>  Artificial Sequence

<400>  9
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Trp Met His Trp Val Arg Gln Ala Pro Gly Gln Gly Leu Glu Trp Met
        35                  40                  45
Gly Arg Ile Gly Pro Asn Ser Gly Phe Thr Ser Tyr Asn Glu Lys Phe
    50                  55                  60
Lys Asn Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Gly Gly Ser Ser Tyr Asp Tyr Phe Asp Tyr Trp Gly Gln Gly
            100                 105                 110
Thr Thr Val Thr Val Ser Ser Ala Ser Thr Lys Gly Pro Ser Val Phe
        115                 120                 125
Pro Leu Ala Pro Cys Ser Arg Ser Thr Ser Glu Ser Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Lys Thr Tyr Thr Cys Asn Val Asp His Lys Pro
        195                 200                 205
Ser Asn Thr Lys Val Asp Lys Arg Val Glu Ser Lys Tyr Gly Pro Pro
    210                 215                 220
Cys Pro Pro Cys Pro Ala Pro Glu Ala Ala Gly Gly Pro Ser Val Phe
225                 230                 235                 240
Leu Phe Pro Pro Lys Pro Lys Asp Thr Leu Met Ile Ser Arg Thr Pro
                245                 250                 255

Glu Val Thr Cys Val Val Val Asp Val Ser Gln Glu Asp Pro Glu Val
            260                 265                 270
Gln Phe Asn Trp Tyr Val Asp Gly Val Glu Val His Asn Ala Lys Thr
        275                 280                 285
Lys Pro Arg Glu Glu Gln Phe Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Gly Leu Pro Ser Ser Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Gln Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Arg Leu Thr Val Asp Lys Ser Arg Trp
                405                 410                 415
Gln Glu Gly Asn Val Phe Ser Cys Ser Val Met His Glu Ala Leu His
            420                 425                 430
Asn His Tyr Thr Gln Lys Ser Leu Ser Leu Ser Leu Gly Lys
        435                 440                 445

<210>  10
<211>  1341
<212>  DNA
<213>  Artificial Sequence

<400>  10
caggtgcaac tggtgcagag cggtgccgag gtgaagaagc ctggcgcaag cgtgaaagtg      60
agctgcaagg ccagcggcta caccttcacc agctactgga tgcactgggt gaggcaggcc     120
cctggacagg gcctggagtg gatgggcagg atcgggccca acagtggttt cactagctac     180
aatgaaaagt tcaagaacag ggtaaccatg accagggaca cctccaccag cacagtgtat     240
atggagctga gcagcctgag gagcgaggac accgccgtgt actactgtgc cagaggcggc     300
agcagctacg actacttcga ctattggggc cagggcacca ccgtgaccgt gagcagtgct     360
tccaccaagg gcccatcggt cttccccctg gcgccctgct ccaggagcac ctccgagagc     420
acagccgccc tgggctgcct ggtcaaggac tacttccccg aaccggtgac ggtgtcgtgg     480
aactcaggcg ccctgaccag cggcgtgcac accttcccgg ctgtcctaca gtcctcagga     540
ctctactccc tcagcagcgt ggtgaccgtg ccctccagca gcttgggcac gaagacctac     600
acctgcaacg tagatcacaa gcccagcaac accaaggtgg acaagagagt tgagtccaaa     660
tatggtcccc catgcccacc atgcccagca cctgaggctg ctgggggacc atcagtcttc     720
ctgttccccc caaaacccaa ggacactctc atgatctccc ggacccctga ggtcacgtgc     780
gtggtggtgg acgtgagcca ggaagacccc gaggtccagt tcaactggta cgtggatggc     840
gtggaggtgc ataatgccaa gacaaagccg cgggaggagc agttcaacag cacgtaccgt     900
gtggtcagcg tcctcaccgt cctgcaccag gactggctga acggcaagga gtacaagtgc     960
aaggtctcca acaaaggcct cccgtcctcc atcgagaaaa ccatctccaa agccaaaggg    1020
cagccccgag agccacaggt gtacaccctg cccccatccc aggaggagat gaccaagaac    1080
caggtcagcc tgacctgcct ggtcaaaggc ttctacccca gcgacatcgc cgtggagtgg    1140

gagagcaatg ggcagccgga gaacaactac aagaccacgc ctcccgtgct ggactccgac 1200
ggctccttct tcctctacag caggctcacc gtggacaaga gcaggtggca ggaggggaat 1260
gtcttctcat gctccgtgat gcatgaggct ctgcacaacc actacacaca gaagagcctc 1320
tccctgtctc tgggtaaatg a 1341

<210> 11
<211> 218
<212> PRT
<213> Artificial Sequence

<400> 11
Asp Ile Val Leu Thr Gln Ser Pro Ala Ser Leu Ala Val Ser Pro Gly
1               5                   10                  15
Gln Arg Ala Thr Ile Thr Cys Arg Ala Ser Glu Ser Val Ser Ile His
            20                  25                  30
Gly Thr His Leu Met His Trp Tyr Gln Gln Lys Pro Gly Gln Pro Pro
        35                  40                  45
Lys Leu Leu Ile Tyr Ala Ala Ser Asn Leu Glu Ser Gly Val Pro Ala
    50                  55                  60
Arg Phe Ser Gly Ser Gly Ser Gly Thr Asp Phe Thr Leu Thr Ile Asn
65                  70                  75                  80
Pro Val Glu Ala Glu Asp Thr Ala Asn Tyr Tyr Cys Gln Gln Ser Phe
                85                  90                  95
Glu Asp Pro Leu Thr Phe Gly Gln Gly Thr Lys Leu Glu Ile Lys Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215

<210> 12
<211> 657
<212> DNA
<213> Artificial Sequence

<400> 12
gacatcgtgc tgacccagag tcccgcctca cttgccgtga gccccggtca gagggccacc 60
atcacctgta gggccagcga gagcgtgagc atccacggca cccacctgat gcactggtat 120
caacagaaac ccggccagcc ccccaaactg ctgatctacg ccgccagcaa cctggagagc 180
ggcgtgcccg ccaggttcag cggctccggc agcggcaccg acttcaccct cactatcaac 240

cccgtggagg ccgaggacac cgccaactac tactgccagc agagcttcga ggaccccctg 300
accttcggcc agggcaccaa gctggagatc aagcgtacgg tggctgcacc atctgtcttc 360
atcttcccgc catctgatga gcagttgaaa tctggaactg cctctgttgt gtgcctgctg 420
aataacttct atcccagaga ggccaaagta cagtggaagg tggataacgc cctccaatcg 480
ggtaactccc aggagagtgt cacagagcag gacagcaagg acagcaccta cagcctcagc 540
agcaccctga cgctgagcaa agcagactac gagaaacaca aagtctacgc ctgcgaagtc 600
acccatcagg gcctgagctc gcccgtcaca aagagcttca acaggggaga gtgttga 657

<210> 13
<211> 232
<212> PRT
<213> Artificial Sequence

<400> 13
Glu Pro Lys Ser Ser Asp Lys Thr His Thr Cys Pro Pro Cys Pro Ala
1               5                   10                  15
Pro Glu Leu Leu Gly Gly Pro Ser Val Phe Leu Phe Pro Pro Lys Pro
            20                  25                  30
Lys Asp Thr Leu Met Ile Ser Arg Thr Pro Glu Val Thr Cys Val Val
        35                  40                  45
Val Asp Val Ser His Glu Asp Pro Glu Val Lys Phe Asn Trp Tyr Val
    50                  55                  60
Asp Gly Val Glu Val His Asn Ala Lys Thr Lys Pro Arg Glu Glu Gln
65                  70                  75                  80
Tyr Asn Ser Thr Tyr Arg Val Val Ser Val Leu Thr Val Leu His Gln
                85                  90                  95
Asp Trp Leu Asn Gly Lys Glu Tyr Lys Cys Lys Val Ser Asn Lys Ala
            100                 105                 110
Leu Pro Ala Pro Ile Glu Lys Thr Ile Ser Lys Ala Lys Gly Gln Pro
        115                 120                 125
Arg Glu Pro Gln Val Tyr Thr Leu Pro Pro Ser Arg Glu Glu Met Thr
    130                 135                 140
Lys Asn Gln Val Ser Leu Thr Cys Leu Val Lys Gly Phe Tyr Pro Ser
145                 150                 155                 160
Asp Ile Ala Val Glu Trp Glu Ser Asn Gly Gln Pro Glu Asn Asn Tyr
                165                 170                 175
Lys Thr Thr Pro Pro Val Leu Asp Ser Asp Gly Ser Phe Phe Leu Tyr
            180                 185                 190
Ser Lys Leu Thr Val Asp Lys Ser Arg Trp Gln Gln Gly Asn Val Phe
        195                 200                 205
Ser Cys Ser Val Met His Glu Ala Leu His Asn His Tyr Thr Gln Lys
    210                 215                 220
Ser Leu Ser Leu Ser Pro Gly Lys
225                 230

<210> 14
<211> 114
<212> PRT
<213> Artificial Sequence

<400>  14
Asn Trp Val Asn Val Ile Ser Asp Leu Lys Lys Ile Glu Asp Leu Ile
1               5                   10                  15
Gln Ser Met His Ile Asp Ala Thr Leu Tyr Thr Glu Ser Asp Val His
            20                  25                  30
Pro Ser Cys Lys Val Thr Ala Met Lys Cys Phe Leu Leu Glu Leu Gln
        35                  40                  45
Val Ile Ser Cys Glu Ser Gly Asp Ala Ser Ile His Asp Thr Val Glu
    50                  55                  60
Asn Leu Ile Ile Leu Ala Asn Asn Ser Leu Ser Ser Asn Gly Asn Val
65                  70                  75                  80
Thr Glu Ser Gly Cys Lys Glu Cys Glu Glu Leu Glu Glu Lys Asn Ile
                85                  90                  95
Lys Glu Phe Leu Gln Ser Phe Val His Ile Val Gln Met Phe Ile Asn
            100                 105                 110
Thr Ser



<210>  15
<211>  417
<212>  PRT
<213>  Artificial Sequence

<400>  15
Ile Thr Cys Pro Pro Pro Met Ser Val Glu His Ala Asp Ile Trp Val
1               5                   10                  15
Lys Ser Tyr Ser Leu Tyr Ser Arg Glu Arg Tyr Ile Cys Asn Ser Gly
            20                  25                  30
Phe Lys Arg Lys Ala Gly Thr Cys Ser Leu Thr Glu Cys Val Leu Asn
        35                  40                  45
Lys Ala Thr Asn Val Ala His Trp Thr Thr Pro Ser Leu Lys Cys Ile
    50                  55                  60
Arg Asp Pro Ala Leu Val His Gln Arg Pro Ala Pro Pro Ser Thr Val
65                  70                  75                  80
Thr Thr Ala Gly Val Thr Pro Gln Pro Glu Ser Leu Ser Pro Ser Gly
                85                  90                  95
Lys Glu Pro Ala Ala Ser Ser Pro Ser Ser Asn Asn Thr Ala Ala Thr
            100                 105                 110
Thr Ala Ala Ile Val Pro Gly Ser Gln Leu Met Pro Ser Lys Ser Pro
        115                 120                 125
Ser Thr Gly Thr Thr Glu Ile Ser Ser His Glu Ser Ser His Gly Thr
    130                 135                 140
Pro Ser Gln Thr Thr Ala Lys Asn Trp Glu Leu Thr Ala Ser Ala Ser
145                 150                 155                 160
His Gln Pro Pro Gly Val Tyr Pro Gln Gly His Ser Asp Thr Thr Gly
                165                 170                 175
Gly Gly Gly Ser Gly Gly Gly Gly Ser Glu Pro Lys Ser Ser Asp Lys
            180                 185                 190
Thr His Thr Cys Pro Pro Cys Pro Ala Pro Glu Leu Leu Gly Gly Pro
        195                 200                 205

Ser Val Phe Leu Phe Pro Pro Lys Pro Lys Asp Thr Leu Met Ile Ser
    210                 215                 220
Arg Thr Pro Glu Val Thr Cys Val Val Val Asp Val Ser His Glu Asp
225                 230                 235                 240
Pro Glu Val Lys Phe Asn Trp Tyr Val Asp Gly Val Glu Val His Asn
                245                 250                 255
Ala Lys Thr Lys Pro Arg Glu Glu Gln Tyr Asn Ser Thr Tyr Arg Val
            260                 265                 270
Val Ser Val Leu Thr Val Leu His Gln Asp Trp Leu Asn Gly Lys Glu
        275                 280                 285
Tyr Lys Cys Lys Val Ser Asn Lys Ala Leu Pro Ala Pro Ile Glu Lys
    290                 295                 300
Thr Ile Ser Lys Ala Lys Gly Gln Pro Arg Glu Pro Gln Val Tyr Thr
305                 310                 315                 320
Leu Pro Pro Ser Arg Glu Glu Met Thr Lys Asn Gln Val Ser Leu Thr
                325                 330                 335
Cys Leu Val Lys Gly Phe Tyr Pro Ser Asp Ile Ala Val Glu Trp Glu
            340                 345                 350
Ser Asn Gly Gln Pro Glu Asn Asn Tyr Lys Thr Thr Pro Pro Val Leu
        355                 360                 365
Asp Ser Asp Gly Ser Phe Phe Leu Tyr Ser Lys Leu Thr Val Asp Lys
    370                 375                 380
Ser Arg Trp Gln Gln Gly Asn Val Phe Ser Cys Ser Val Met His Glu
385                 390                 395                 400
Ala Leu His Asn His Tyr Thr Gln Lys Ser Leu Ser Leu Ser Pro Gly
                405                 410                 415
Lys

<210>  16
<211>  417
<212>  PRT
<213>  Artificial Sequence

<400>  16
Glu Pro Lys Ser Ser Asp Lys Thr His Thr Cys Pro Pro Cys Pro Ala
1               5                   10                  15
Pro Glu Leu Leu Gly Gly Pro Ser Val Phe Leu Phe Pro Pro Lys Pro
            20                  25                  30
Lys Asp Thr Leu Met Ile Ser Arg Thr Pro Glu Val Thr Cys Val Val
        35                  40                  45
Val Asp Val Ser His Glu Asp Pro Glu Val Lys Phe Asn Trp Tyr Val
    50                  55                  60
Asp Gly Val Glu Val His Asn Ala Lys Thr Lys Pro Arg Glu Glu Gln
65                  70                  75                  80
Tyr Asn Ser Thr Tyr Arg Val Val Ser Val Leu Thr Val Leu His Gln
                85                  90                  95
Asp Trp Leu Asn Gly Lys Glu Tyr Lys Cys Lys Val Ser Asn Lys Ala
            100                 105                 110
Leu Pro Ala Pro Ile Glu Lys Thr Ile Ser Lys Ala Lys Gly Gln Pro

115                 120                 125
Arg Glu Pro Gln Val Tyr Thr Leu Pro Pro Ser Arg Glu Glu Met Thr
    130                 135                 140
Lys Asn Gln Val Ser Leu Thr Cys Leu Val Lys Gly Phe Tyr Pro Ser
145                 150                 155                 160
Asp Ile Ala Val Glu Trp Glu Ser Asn Gly Gln Pro Glu Asn Asn Tyr
                165                 170                 175
Lys Thr Thr Pro Pro Val Leu Asp Ser Asp Gly Ser Phe Phe Leu Tyr
            180                 185                 190
Ser Lys Leu Thr Val Asp Lys Ser Arg Trp Gln Gln Gly Asn Val Phe
        195                 200                 205
Ser Cys Ser Val Met His Glu Ala Leu His Asn His Tyr Thr Gln Lys
    210                 215                 220
Ser Leu Ser Leu Ser Pro Gly Lys Gly Gly Gly Gly Ser Gly Gly Gly
225                 230                 235                 240
Gly Ser Ile Thr Cys Pro Pro Pro Met Ser Val Glu His Ala Asp Ile
                245                 250                 255
Trp Val Lys Ser Tyr Ser Leu Tyr Ser Arg Glu Arg Tyr Ile Cys Asn
            260                 265                 270
Ser Gly Phe Lys Arg Lys Ala Gly Thr Cys Ser Leu Thr Glu Cys Val
        275                 280                 285
Leu Asn Lys Ala Thr Asn Val Ala His Trp Thr Thr Pro Ser Leu Lys
    290                 295                 300
Cys Ile Arg Asp Pro Ala Leu Val His Gln Arg Pro Ala Pro Pro Ser
305                 310                 315                 320
Thr Val Thr Thr Ala Gly Val Thr Pro Gln Pro Glu Ser Leu Ser Pro
                325                 330                 335
Ser Gly Lys Glu Pro Ala Ala Ser Ser Pro Ser Ser Asn Asn Thr Ala
            340                 345                 350
Ala Thr Thr Ala Ala Ile Val Pro Gly Ser Gln Leu Met Pro Ser Lys
        355                 360                 365
Ser Pro Ser Thr Gly Thr Thr Glu Ile Ser Ser His Glu Ser Ser His
    370                 375                 380
Gly Thr Pro Ser Gln Thr Thr Ala Lys Asn Trp Glu Leu Thr Ala Ser
385                 390                 395                 400
Ala Ser His Gln Pro Pro Gly Val Tyr Pro Gln Gly His Ser Asp Thr
                405                 410                 415
Thr


<210>  17
<211>  315
<212>  PRT
<213>  Artificial Sequence

<400>  17
Ile Thr Cys Pro Pro Pro Met Ser Val Glu His Ala Asp Ile Trp Val
1               5                   10                  15
Lys Ser Tyr Ser Leu Tyr Ser Arg Glu Arg Tyr Ile Cys Asn Ser Gly
            20                  25                  30

Phe Lys Arg Lys Ala Gly Thr Cys Ser Leu Thr Glu Cys Val Leu Asn
        35                  40                  45
Lys Ala Thr Asn Val Ala His Trp Thr Thr Pro Ser Leu Lys Cys Ile
    50                  55                  60
Arg Asp Pro Ala Leu Val His Gln Arg Gly Gly Gly Gly Ser Gly Gly
65                  70                  75                  80
Gly Gly Ser Glu Pro Lys Ser Ser Asp Lys Thr His Thr Cys Pro Pro
                85                  90                  95
Cys Pro Ala Pro Glu Leu Leu Gly Gly Pro Ser Val Phe Leu Phe Pro
            100                 105                 110
Pro Lys Pro Lys Asp Thr Leu Met Ile Ser Arg Thr Pro Glu Val Thr
        115                 120                 125
Cys Val Val Val Asp Val Ser His Glu Asp Pro Glu Val Lys Phe Asn
    130                 135                 140
Trp Tyr Val Asp Gly Val Glu Val His Asn Ala Lys Thr Lys Pro Arg
145                 150                 155                 160
Glu Glu Gln Tyr Asn Ser Thr Tyr Arg Val Val Ser Val Leu Thr Val
                165                 170                 175
Leu His Gln Asp Trp Leu Asn Gly Lys Glu Tyr Lys Cys Lys Val Ser
            180                 185                 190
Asn Lys Ala Leu Pro Ala Pro Ile Glu Lys Thr Ile Ser Lys Ala Lys
        195                 200                 205
Gly Gln Pro Arg Glu Pro Gln Val Tyr Thr Leu Pro Pro Ser Arg Glu
    210                 215                 220
Glu Met Thr Lys Asn Gln Val Ser Leu Thr Cys Leu Val Lys Gly Phe
225                 230                 235                 240
Tyr Pro Ser Asp Ile Ala Val Glu Trp Glu Ser Asn Gly Gln Pro Glu
                245                 250                 255
Asn Asn Tyr Lys Thr Thr Pro Pro Val Leu Asp Ser Asp Gly Ser Phe
            260                 265                 270
Phe Leu Tyr Ser Lys Leu Thr Val Asp Lys Ser Arg Trp Gln Gln Gly
        275                 280                 285
Asn Val Phe Ser Cys Ser Val Met His Glu Ala Leu His Asn His Tyr
    290                 295                 300
Thr Gln Lys Ser Leu Ser Leu Ser Pro Gly Lys
305                 310                 315

<210>  18
<211>  315
<212>  PRT
<213>  Artificial Sequence

<400>  18
Glu Pro Lys Ser Ser Asp Lys Thr His Thr Cys Pro Pro Cys Pro Ala
1               5                   10                  15
Pro Glu Leu Leu Gly Gly Pro Ser Val Phe Leu Phe Pro Pro Lys Pro
            20                  25                  30
Lys Asp Thr Leu Met Ile Ser Arg Thr Pro Glu Val Thr Cys Val Val
        35                  40                  45
Val Asp Val Ser His Glu Asp Pro Glu Val Lys Phe Asn Trp Tyr Val

50                  55                  60
Asp Gly Val Glu Val His Asn Ala Lys Thr Lys Pro Arg Glu Glu Gln
65                  70                  75                  80
Tyr Asn Ser Thr Tyr Arg Val Val Ser Val Leu Thr Val Leu His Gln
                85                  90                  95
Asp Trp Leu Asn Gly Lys Glu Tyr Lys Cys Lys Val Ser Asn Lys Ala
            100                 105                 110
Leu Pro Ala Pro Ile Glu Lys Thr Ile Ser Lys Ala Lys Gly Gln Pro
        115                 120                 125
Arg Glu Pro Gln Val Tyr Thr Leu Pro Pro Ser Arg Glu Glu Met Thr
    130                 135                 140
Lys Asn Gln Val Ser Leu Thr Cys Leu Val Lys Gly Phe Tyr Pro Ser
145                 150                 155                 160
Asp Ile Ala Val Glu Trp Glu Ser Asn Gly Gln Pro Glu Asn Asn Tyr
                165                 170                 175
Lys Thr Thr Pro Pro Val Leu Asp Ser Asp Gly Ser Phe Phe Leu Tyr
            180                 185                 190
Ser Lys Leu Thr Val Asp Lys Ser Arg Trp Gln Gln Gly Asn Val Phe
        195                 200                 205
Ser Cys Ser Val Met His Glu Ala Leu His Asn His Tyr Thr Gln Lys
    210                 215                 220
Ser Leu Ser Leu Ser Pro Gly Lys Gly Gly Gly Gly Ser Gly Gly Gly
225                 230                 235                 240
Gly Ser Ile Thr Cys Pro Pro Pro Met Ser Val Glu His Ala Asp Ile
                245                 250                 255
Trp Val Lys Ser Tyr Ser Leu Tyr Ser Arg Glu Arg Tyr Ile Cys Asn
            260                 265                 270
Ser Gly Phe Lys Arg Lys Ala Gly Thr Cys Ser Leu Thr Glu Cys Val
        275                 280                 285
Leu Asn Lys Ala Thr Asn Val Ala His Trp Thr Thr Pro Ser Leu Lys
    290                 295                 300
Cys Ile Arg Asp Pro Ala Leu Val His Gln Arg
305                 310                 315

## Description

### FIELD OF THE INVENTION

The present disclosure belongs to the field of medicine, and relates to use of IL-15 protein complex in combination with PD-L1 antibody in the preparation of medicament for the prevention or treatment of tumor diseases.

### BACKGROUND OF THE INVENTION

Tumor immunotherapy is a hot topic in the field of tumor therapy in recent years, and tumor immunotherapy with T cells is critical to tumor immunotherapy. Tumor immunotherapy plays a killing effect on tumors by fully utilizing and mobilizing the killer T cells present in tumor-bearing subjects. It may be the most effective and safest way for the treatment of tumors. Tumor escape is a huge obstacle to tumor immunotherapy. Tumor cells utilize their own inhibitory effect on the immune system to promote the uncontrolledgrowth of tumors. There is an extremely complex relationship between the mechanism underlying the tumor immune escape and the body's immune response to tumors. At the early stage of tumor immunotherapy, the tumor-specific killer T cells still keep biological activity. However, the tumor-specific killer T cells lose their killing function at the advanced stage, as the tumor grows. Therefore, tumor immunotherapy currently is to maximize the subject's own immune system response to tumors. The key point for the tumor immunotherapy is not only to activate the *in vivo* innate immune system response, but also to maintain the duration and the intensity of the immune system response.

Programmed death receptor 1 (PD-1) antibodies can specifically recognize and bind to PD-1 present on the surface of lymphocytes, block the PD-1/PD-L1 signaling pathway, thereby activate the killing effect of immune T cells on tumors, and mobilize the body's immune system to eliminate tumor cells from the body. There are two PD-1 ligands, PD-L1 and PD-L2. PD-L1 is mainly expressed on T cells, B cells, macrophages and dendritic cells (DCs), and the expression on cells can be up-regulated upon activation. PD-L1 inhibits the immune system by binding to PD-1 and B7-1. Various tumor cells and immune cells present in the tumor tissue microenvironment express PD-L1. Recent studies have found that high expression level of PD-L1 protein was detected in human tumor tissues, such as breast cancer, lung cancer, gastric cancer, intestinal cancer, kidney cancer, melanoma, non-small cell lung cancer, colon cancer, bladder cancer, ovarian cancer, pancreatic cancer and liver cancer, and the expression level of PD-L1 is closely associated with the clinical and prognosis of patients. PD-L1 acts as a second signaling pathway to inhibit T cell proliferation. Therefore, blocking the binding between PD-L1/PD-1 has become a very potential emerging target in the field of tumor immunotherapy. The combination therapy of immune checkpoint inhibitors (such as PD-1 and PD-L1 antibodies) with other agents is also a hot research field. Currently, a series of anti-PD-L1 antibodies have been reported, among them, the PD-L1 antibodies disclosed in WO2017084495 can effectively improve the effect of suppressing the tumorigenesis and development.

Interleukin 15 (IL-15) is a cytokine of approximately 12-14kD, discovered by Grabstein et al. in 1994. IL-15 plays a role in the body's normal immune response, for example, promoting the proliferation of T cells, B cells, and natural killer (NK) cells. IL-15 exerts its biological activity by binding to its receptor. The IL-15 receptor is composed of three receptor subunits: IL-15 receptor α (IL-15Rα), IL-2 receptor β (IL-2Rβ) and γc. IL-15Rα comprises a Sushi domain, which can bind to IL-15 and is necessary for the bound IL-15 to exert the biological functions. In recent years, it has been found that the complex formed by IL-15 and its receptor IL-15Rα can significantly enhance the biological activity of IL-15.

Many domestic or foreign companies or research institutions were engaged in IL-15 immunotherapy related research due to the positive anticipation of IL-15 in the field of tumor immunotherapy. For example, the IL-15-hIgG4Fc homodimer involved in the patent CN100334112C is useful for the treatment of microbial infections; IL-15N72D:IL-15RαSu/Fc fusion protein complex is disclosed in CN103370339B, wherein the IL-15 polypeptide thereof has N72D mutation, and the mutant shows decreased binding activity to IL-15βγC receptor when compared to the original IL-15; IL-15 protein complex disclosed in WO2016095642 is composed of an IL-15 polypeptide and an IL-15Rα/Fc, wherein disulfide bond(s) is(are) introduced between IL-15 and IL-15Rα, which can not only improve the molecular stability and biological activity, but also simplify the preparation process.

At present, some researches have been carried out on the combined therapy of immune checkpoint inhibitors and IL-15. John M Wrangle et al. (The Lancet Oncology, Volume 19, Issue 5, May 2018, Pages 694-704) have evaluated the safety of IL-15 complex ALT-803 in combination with anti-PD-1 antibody nivolumab for the treatment of advanced non-squamous non-small cell lung cancer.

The present disclosure provides use of an IL-15 protein complex in combination with a PD-L1 antibody in the preparation of medicament for the prevention or treatment of tumor diseases, which shows a favorable anti-tumor effect.

### SUMMARY OF THE INVENTION

The present disclosure provides use of an IL-15 or protein complex thereof in combination with a PD-L1 antibody or antigen-binding fragment thereof in the preparation of medicament for the prevention or treatment of tumor diseases.

In some embodiments, any one of the PD-L1 antibody or antigen-binding fragment thereof comprises CDR(s) selected from the following CDR region sequences or mutant sequences thereof: antibody heavy chain variable region HCDR region sequences: SEQ ID NOs: 1-3; and antibody light chain variable region LCDR region sequences: SEQ ID NOs: 4-6;

In particular:

| | |
|---|---|
| HCDR1 is selected from: SYWMH | SEQ ID NO: 1 |
| HCDR2 is selected from: RI X₁PNSG X₂TSYNEKFKN | SEQ ID NO: 2 |
| HCDR3 is selected from: GGSSYDYFDY | SEQ ID NO: 3 |
| LCDR1 is selected from: RASESVSIHGTHLMH | SEQ ID NO: 4 |
| LCDR2 is selected from: AASNLES | SEQ ID NO: 5 |
| LCDR3 is selected from: QQSFEDPLT | SEQ ID NO: 6; |

wherein X₁ is selected from H or G, preferably G; X ₂ is selected from G or F, preferably F.

In some embodiments, the PD-L1 antibody or antigen-binding fragment thereof comprises the light chain variable region CDR sequences having at least 85%, 86%, 87%, 88%, 89%, 90%, 91%, 92%, 93%, 94%, 95%, 96%, 97%, 98%, or 99% sequence identity to amino acid sequences: SEQ ID NO: 4, SEQ ID NO: 5 and SEQ ID NO: 6; and the heavy chain variable region CDR sequences having at least 85%, 86%, 87%, 88%, 89%, 90%, 91%, 92%, 93%, 94%, 95%, 96%, 97%, 98%, or 99% sequence identity to amino acid sequences: SEQ ID NO: 1, SEQ ID NO: 2 and SEQ ID NO: 3.

In some embodiments, the PD-L1 antibody or antigen-binding fragment thereof may be selected from the group consisting of a murine antibody, a chimeric antibody, a humanized antibody and a human antibody, preferably a humanized antibody.

In some embodiments, the PD-L1 antibody or antigen-binding fragment thereof comprises a heavy chain variable region sequence having at least 85%, 86%, 87%, 88%, 89%, 90%, 91%, 92%, 93%, 94%, 95%, 96%, 97%, 98%, or 99% sequence identity to amino acid sequence SEQ ID NO: 7; and a light chain variable region sequence having at least 85%, 86%, 87%, 88%, 89%, 90%, 91%, 92%, 93%, 94%, 95%, 96%, 97%, 98%, or 99% sequence identity to amino acid sequence SEQ ID NO: 8.

In some embodiments, the PD-L1 antibody or antigen-binding fragment thereof further comprises a heavy chain constant region of human IgG1, IgG2, IgG3 or IgG4 or a variant thereof, preferably comprises a heavy chain constant region of human IgG2 or IgG4, more preferably comprises a heavy chain constant region of IgG4 into which F234A and L235A mutations are introduced; and the humanized antibody light chain further comprises a constant region of human kappa, lambda chain or a variant thereof.

In some embodiments, the PD-L1 antibody or antigen-binding fragment thereof comprises a heavy chain variable region sequence having at least 85%, 86%, 87%, 88%, 89%, 90%, 91%, 92%, 93%, 94%, 95%, 96%, 97%, 98%, or 99% sequence identity to amino acid sequence SEQ ID NO: 9; and a light chain sequence having at least 85%, 86%, 87%, 88%, 89%, 90%, 91%, 92%, 93%, 94%, 95%, 96%, 97%, 98%, or 99% sequence identity to amino acid sequence SEQ ID NO: 11.

In some embodiments, the heavy chain sequence of the PD-L1 antibody or antigen-binding fragment thereof is SEQ ID NO: 9, and the light chain sequence is SEQ ID NO: 11.

QVQLVQSGAEVKKPGASVKVSCKASGYTFTSYWMHWVRQAPGQGLEWMGRIGPNSGFTS
YNEKFKNRVTMTRDTSTSTVYMELSSLRSEDTAVYYCARGGSSYDYFDYWGQGTTVTVSS
SEQ ID NO: 7;

DIVLTQSPASLAVSPGQRATITCRASESVSIHGTHLMHWYQQKPGQPPKLLIYAASNLESG
VPARFSGSGSGTDFTLTINPVEAEDTANYYCQQSFEDPLTFGQGTKLEIK
SEQ ID NO: 8;

Note: The italic sequences represent FRs, and the underlined sequences represent CDRs.

Heavy chain sequence

QVQLVQSGAEVKKPGASVKVSCKASGYTFTSYWMHWVRQAPGQGLEWMGRIGPNS
GFTSYNEKFKNRVTMTRDTSTSTVYMELSSLRSEDTAVYYCARGGSYDYFDYWGQ
GTTVTVSSASTKGPSVFPLAPCSRSTSESTAALGCLVKDYFPEPVTVSWNSGALTSGVH
TFPAVLQSSGLYSLSSVVTVPSSSLGTKTYTCNVDHKPSNTKVDKRVESKYGPPCPPCP
APEAAGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPRE
PQVYTLPPSQEEMTKNQVSLTCLVKGFYPSDIAVEWESNGQPENNYKTTPPVLDSDGS
FFLYSRLTVDKSRWQEGNVFSCSVMHEALHNHYTQKSLSLSLGK
SEQ ID NO: 9

Gene sequence encoding the heavy chain sequence

CAGGTGCAACTGGTGCAGAGCGGTGCCGAGGTGAAGAAGCCTGGCGCAAGCGTG
AAAGTGAGCTGCAAGGCCAGCGGCTACACCTTCACCAGCTACTGGATGCACTGGG
TGAGGCAGGCCCCTGGACAGGGCCTGGAGTGGATGGGCAGGATCGGCCCAACA
GTGGTTTCACTAGCTACAATGAAAAGTTCAAGAACAGGGTAACCATGACCAGGGA
CACCTCCACCAGCACAGTGTATATGGAGCTGAGCAGCCTGAGGAGCGAGGACACC
GCCGTGTACTACTGTGCCAGAGGCGGCAGCAGCTACGACTACTTCGACTATTGGG
GCCAGGGCACCACCGTGACCGTGAGCAGTGCTTCCACCAAGGGCCCATCGGTCTT
CCCCCTGGCGCCCTGCTCCAGGAGCACCTCCGAGAGCACAGCCGCCCTGGGCTGC
CTGGTCAAGGACTACTTCCCCGAACCGGTGACGGTGTCGTGGAACTCAGGCGCCCT
GACCAGCGGCGTGCACACCTTCCCGGCTGTCCTACAGTCCTCAGGACTCTACTCCC
TCAGCAGCGTGGTGACCGTGCCCTCCAGCAGCTTGGGCACGAAGACCTACACCTG
CAACGTAGATCACAAGCCCAGCAACACCAAGGTGGACAAGAGAGTTGAGTCCAA
ATATGGTCCCCCATGCCCACCATGCCCAGCACCTGAGGCTGCTGGGGGACCATCA

GTCTTCCTGTTCCCCCCAAAACCCAAGGACACTCTCATGATCTCCCGGACCCCTGA
GGTCACGTGCGTGGTGGTGGACGTGAGCCAGGAAGACCCCGAGGTCCAGTTCAAC
TGGTACGTGGATGGCGTGGAGGTGCATAATGCCAAGACAAAGCCGCGGGAGGAG
CAGTTCAACAGCACGTACCGTGTGGTCAGCGTCCTCACCGTCCTGCACCAGGACTG
GCTGAACGGCAAGGAGTACAAGTGCAAGGTCTCCAACAAAGGCCTCCCGTCCTCC
ATCGAGAAAACCATCTCCAAAGCCAAAGGGCAGCCCCGAGAGCCACAGGTGTACA
CCCTGCCCCCATCCCAGGAGGAGATGACCAAGAACCAGGTCAGCCTGACCTGCCT
GGTCAAAGGCTTCTACCCCAGCGACATCGCCGTGGAGTGGGAGAGCAATGGGCAG
CCGGAGAACAACTACAAGACCACGCCTCCCGTGCTGGACTCCGACGGCTCCTTCTT
CCTCTACAGCAGGCTCACCGTGGACAAGAGCAGGTGGCAGGAGGGGAATGTCTTC
TCATGCTCCGTGATGCATGAGGCTCTGCACAACCACTACACACAGAAGAGCCTCTC
CCTGTCTCTGGGTAAATGA
SEQ ID NO: 10

Light chain sequence

DIVLTQSPASLAVSPGQRATITCRASESVSIHGTHLMHWYQQKPGQPPKLLIYAASNLE
SGVPARFSGSGSGTDFTLTINPVEAEDTANYYCQQSFEDPLTFGQGTKLEIKRTVAAPS
VFIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC
SEQ ID NO: 11

Gene sequence encoding the light chain sequence:

GACATCGTGCTGACCCAGAGTCCCGCCTCACTTGCCGTGAGCCCCGGTCAGAGGG
CCACCATCACCTGTAGGGCCAGCGAGAGCGTGAGCATCCACGGCACCCACCTGAT
GCACTGGTATCAACAGAAACCCGGCCAGCCCCCCAAACTGCTGATCTACGCCGCC
AGCAACCTGGAGAGCGGCGTGCCCGCCAGGTTCAGCGGCTCCGGCAGCGGCACCG
ACTTCACCCTCACTATCAACCCCGTGGAGGCCGAGGACACCGCCAACTACTACTGC
CAGCAGAGCTTCGAGGACCCCCTGACCTTCGGCCAGGGCACCAAGCTGGAGATCA
AGCGTACGGTGGCTGCACCATCTGTCTTCATCTTCCCGCCATCTGATGAGCAGTTG
AAATCTGGAACTGCCTCTGTTGTGTGCCTGCTGAATAACTTCTATCCCAGAGAGGC
CAAAGTACAGTGGAAGGTGGATAACGCCCTCCAATCGGGTAACTCCCAGGAGAGT
GTCACAGAGCAGGACAGCAAGGACAGCACCTACAGCCTCAGCAGCACCCTGACGC
TGAGCAAAGCAGACTACGAGAAACACAAAGTCTACGCCTGCGAAGTCACCCATCA
GGGCCTGAGCTCGCCCGTCACAAAGAGCTTCAACAGGGGAGAGTGTTGA
SEQ ID NO: 12

In some embodiments, the IL-15 protein complex is composed of a soluble fusion protein (I) and a soluble fusion protein (II); wherein the soluble fusion protein (I) comprises an IL-15 polypeptide or functional fragment thereof; the soluble fusion protein (II) comprises an IL-15Rα polypeptide or functional fragment thereof; one or more amino acid sites of the soluble fusion protein (I) or the soluble fusion protein (II) is/are mutated into Cys, to form disulfide bond(s) by pairing with Cys at the corresponding amino acid site(s) of the soluble fusion protein (II) or soluble fusion protein (I).

In some embodiments, wherein the soluble fusion protein (II) further comprises an Fc fragment or a mutant thereof; preferably, the soluble fusion protein (II) is composed of an IL-15Rα polypeptide or functional fragment thereof connected to the N-terminus of the Fc fragment; more preferably, the Fc fragment is SEQ ID NO: 13.

EPKSSDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPA
PIEKTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSPG
K
SEQ ID NO: 13.

In some embodiments, the sequence of the soluble fusion protein (I) is SEQ ID NO: 14.

NWVNVISDLKKIEDLIQSMHIDATLYTESDVHPSCKVTAMKCFLLELQVISCESGDASI
HDTVENLIILANNSLSSNGNVTESGCKECEELEEKNIKEFLQSFVHIVQMFINTS
SEQ ID NO: 14

In certain embodiments, the amino acid Cys mutation(s) of the IL-15 protein complex occur(s) at L45, Q48, V49, L52, E53, C88 or E89 of the IL-15 polypeptide or functional fragment thereof, preferably occur(s) at L52, E53 or E89, more preferably at L52.

In certain embodiments, the amino acid Cys mutation(s) of the IL-15 protein complex occur(s) at K34, L42, A37, G38 or S40 of the IL-15Rα polypeptide or functional fragment thereof, preferably occur(s) at A37, G38 or S40, more preferably at S40.

In some embodiments, the sequence of the soluble fusion protein (II) is selected from the group consisting of SEQ ID NO: 15, SEQ ID NO: 16, SEQ ID NO: 17 and SEQ ID NO: 18.

ITCPPPMSVEHADIWVKSYSLYSRERYICNSGFKRKAGTCSLTECVLNKATNVAHWTT
PSLKCIRDPALVHQRPAPPSTVTTAGVTPQPESLSPSGKEPAASSPSSNNTAATTAAIVP
GSQLMPSKSPSTGTTEISSHESSHGTPSQTTAKNWELTASASHQPPGVYPQGHSDTTGG
GGSGGGGSEPKSSDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVMHEALHNHY
TQKSLSLSPGK
SEQ ID NO: 15

EPKSSDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPA
PIEKTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSPG
KGGGGSGGGGSITCPPPMSVEHADIWVKSYSLYSRERYICNSGFKRKAGTCSLTECVL
NKATNVAHWTTPSLKCIRDPALVHQRPAPPSTVTTAGVTPQPESLSPSGKEPAASSPSS
NNTAATTAAIVPGSQLMPSKSPSTGTTEISSHESSHGTPSQTTAKNWELTASASHQPPG
VYPQGHSDTT
SEQ ID NO: 16

ITCPPPMSVEHADIWVKSYSLYSRERYICNSGFKRKAGTCSLTECVLNKATNVAHWTT
PSLKCIRDPALVHQRGGGGSGGGGSEPKSSDKTHTCPPCPAPELLGGPSVFLFPPKPKD
TLMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPSREEMTKNQVS
LTCLVKGFYPSDIAVEWESNGQPENNYKTTPPVLDSDGSFFLYSKLTVDKSRWQQGN
VFSCSVMHEALHNHYTQKSLSLSPGK
SEQ ID NO: 17

EPKSSDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPA
PIEKTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSPG
KGGGGSGGGGSITCPPPMSVEHADIWVKSYSLYSRERYICNSGFKRKAGTCSLTECVL

NKATNVAHWTTPSLKCIRDPALVHQR
SEQ ID NO: 18

In some embodiments, the IL-15 protein complex is selected from the following combinations of the soluble fusion protein (I) and soluble fusion protein (II):

| No. | Soluble fusion protein (I) | Soluble fusion protein (II) |
|---|---|---|
| 1 | IL-15(L52C)(SEQ ID NO: 14) | IL-15Rα-ECD(S40C)-Fc(SEQ ID NO: 15) |
| 2 | IL-15(L52C)(SEQ ID NO: 14) | Fc-IL-15Rα-ECD(S40C)(SEQ ID NO: 16) |
| 3 | IL-15(L52C)(SEQ ID NO: 14) | IL-15Rα-Sushi+(S40C)-Fc(SEQ ID NO:17) |
| 4 | IL-15(L52C)(SEQ ID NO: 14) | Fc-IL-15Rα-sushi+(S40C)(SEQ ID NO:18). |

In some embodiments, the IL-15 protein complex is selected from the combination of IL-15 (L52C) (SEQ ID NO: 14) soluble fusion protein (I) and IL-15Rα-Sushi+ (S40C)-Fc (SEQ ID NO: 17) soluble fusion protein (II).

In some embodiments, the tumor is selected from the group consisting of malignant tumor and benign tumor. The malignant tumor is selected from the group consisting of melanoma, skin cancer, renal cell carcinoma, liver cancer, gastric cancer, breast cancer, colorectal cancer, glioblastoma, ovarian cancer, prostate cancer, hematologic malignancy, urothelial/bladder cancer, lung cancer, esophageal cancer, and head and neck cancer.

The hematologic malignancy described in the present disclosure includes, but is not limited to, acute and chronic myelogenous leukemia, acute lymphocytic leukemia, chronic lymphocytic leukemia, bone marrow tissue proliferative disease, multiple myeloma, Hodgkin's disease, Non-Hodgkin's lymphoma, B-cell lymphoma, T-cell lymphoma, follicular center cell lymphoma and chronic granulocytic leukemia.

In some embodiments, the tumor is selected from the group consisting of advanced tumors, relapsed and refractory tumors, tumors that experienced failed treatment of chemotherapy agent and/or relapsed, tumors that experienced failed radiotherapy and/or relapsed, tumors that experienced failed therapy of targeted agent and/or relapsed, and tumors that experienced failed immunotherapy and/or relapsed.

In certain embodiments, the tumor is selected from advanced/metastatic malignancies.

In some embodiments, the dose of the IL-15 or protein complex thereof is selected from 1-100µg/kg, preferably selected from the group consisting of 1µg/kg, 2µg/kg, 3µg/kg, 4µg/kg, 5µg/kg, 6µg/kg, 7µg/kg, 8µg/kg, 9µg/kg, 10µg/kg, 11µg/kg, 12µg/kg, 13µg/kg, 14µg/kg, 15µg/kg, 16µg/kg, 17µg/kg, 18µg/kg, 19µg/kg, 20µg/kg, 21µg/kg, 22µg/kg, 23µg/kg, 24µg/kg and 25µg/kg; preferably, 1µg/kg, 3µg/kg, 6µg/kg, 10µg/kg, 15µg/kg, or 20µg/kg.

In some embodiments, the dose of the PD-L1 antibody or antigen-binding fragment thereof is selected from 50-3000 mg, preferably 490-2000 mg, more preferably 490 mg, 500 mg, 550 mg, 600 mg, 750 mg, 1200 mg, 1280 mg, or 1500 mg, most preferably, 600 mg or 750 mg.

The combined administration route of the present disclosure is selected from oral administration, parenteral administration, and transdermal administration. The parenteral administration includes, but is not limited to, intravenous injection, subcutaneous injection, and intramuscular injection.

The present disclosure further relates to use of an IL-15 or protein complex thereof in combination with PD-L1 antibody or antigen-binding fragment thereof in the preparation of medicament for the prevention or treatment of tumor diseases, wherein the dosing frequency of the IL-15 or protein complex thereof may be once a week, once every two weeks, or once every three weeks. The dosing frequency of the PD-L1 antibody or antigen-binding fragment thereof may be once a week, once every two weeks, once every three weeks, or once every four weeks.

In some embodiments, for the IL-15 or protein complex thereof, the dosing frequency is once a week, and the dose is 1µg/kg, 3µg/kg, 6µg/kg, 10µg/kg, 15µg/kg or 20µg/ kg; for the PD-L1 antibody or antigen-binding fragment thereof, the dosing frequency is once every 2 weeks, and the dose is 600mg or 750mg.

In some embodiments, the IL-15 or protein complex thereof is administrated as a single-agent loading, and then administrated in combination with the PD-L1 antibody or antigen-binding fragment thereof. The loading period may be 1 week, 2 weeks, 3 weeks, 4 weeks, 5 weeks or 6 weeks, preferably 2 weeks or 4 weeks. The dose may be 1 µg/kg, 3 µg/kg, 6 µg/kg, 10 µg/kg, 15 µg/kg or 20 µg/kg each time, and the dosing frequency may be once a week.

In some embodiments, the present disclosure further relates to use of the IL-15 or protein complex thereof in combination with the PD-L1 antibody or antigen-binding fragment thereof in the preparation of medicament for the prevention or treatment of tumor diseases, wherein the IL-15 or protein complex thereof is administrated firstly as a single drug for 2 or 4 weeks, the dose may be 1µg/kg, 3µg/kg, 6µg/kg, 10µg/kg, 15µg/kg or 20µg/kg each time, and the dosing frequency is once a week; and then the IL-15 or protein complex thereof is administrated in combination with the PD-L1 antibody or antigen-binding fragment thereof, the dosing frequency of the IL-15 or protein complex thereof may be once a week, the dose may be 1µg/kg, 3µg/kg, 6µg/kg, 10µg/kg, 15µg/kg or 20µg/kg each time, and the dosing frequency of the PD-L1 antibody or antigen-binding fragment thereof is once every 2 weeks or once every 3 weeks, the dose is 600mg or 750mg.

In the present disclosure, the dosing period can be 1 day, 3 days, 1 week, 2 weeks, 3 weeks (21 days), 3-4 weeks (21-28 days), 4 weeks (28 days), 5 weeks, or 6 weeks.

In some embodiments, the dosing period is 4 weeks, wherein the first 2 weeks are deemed as loading period, the IL-15 or protein complex thereof is administrated on days 1, 8, 15 and 22 in each dosing period; and the PD-L1 antibody or antigen-binding fragment thereof is administrated on day 15 in each dosing period.

In some embodiments, the dosing period is 4 weeks, the IL-15 or protein complex thereof is administrated on days 1, 8, 15 and 22 in each dosing period; and the PD-L1 antibody or antigen-binding fragment thereof is administrated on days 1 and 15 in each dosing period.

In some embodiments, the dosing period is 5 weeks, the IL-15 or protein complex thereof is administrated on days 1, 8, 15 and 22 in each dosing period; and the PD-L1 antibody or antigen-binding fragment thereof is administrated on days 1 and 15 in each dosing period.

In some embodiments, the dosing period is 6 weeks, the IL-15 or protein complex thereof is administrated on days 1, 8, 15 and 22 in each dosing period; and the PD-L1 antibody or antigen-binding fragment thereof is administrated on days 1 and 15 in each dosing period.

In some embodiments, the dosing period is 6 weeks, the IL-15 or protein complex thereof is administrated on days 1, 8, 15, 22 and 29 in each dosing period; and the PD-L1 antibody or antigen-binding fragment thereof is administrated on days 1 and 15 in each dosing period.

In some embodiments, the PD-L1 antibody or antigen-binding fragment thereof is administrated by injection, such as subcutaneous or intravenous injection.

In some embodiments, the IL-15 or protein complex thereof is administrated by injection, such as subcutaneous or intravenous injection, preferably subcutaneous administration.

In some embodiments, where administration of the IL-15 or protein complex thereof, and the PD-L1 antibody or antigen-binding fragment thereof results in intolerance in patients due to the toxic and side effects, one can appropriately modify the dose and dosing frequency of the agents.

In the embodiments described in the present disclosure, the combined administration optionally further includes other components, and the other components include but are not limited to other anti-tumor agents and the like.

The present disclosure also provides a method for treating tumor diseases, including administrating the IL-15 or protein complex thereof and the PD-L1 antibody or antigen-binding fragment thereof to a patient.

In some embodiments, the patient experienced failed standard treatment or was intolerant to the standard treatment, or there was no more standard treatment once the disease relapsed/progressed.

The present disclosure also provides a method for treating tumor diseases, including administrating a PD-L1 antibody or antigen-binding fragment thereof to a patient.

The PD-L1 antibody or antigen-binding fragment thereof can be administrated alone or in combination with other agents (for example, chemotherapy agents).

Wherein, the PD-L1 antibody or antigen-binding fragment thereof is the PD-L1 antibody or antigen-binding fragment thereof described above.

In some embodiments, the method includes administrating a fixed dose of the PD-L1 antibody or antigen-binding fragment thereof to the patient.

The dose administrated is not related to the weight of the patient.

The fixed dose of the PD-L1 antibody or antigen-binding fragment thereof is selected from 490-2000 mg, preferably 490 mg, 500 mg, 550 mg, 600 mg, 750 mg, 1200 mg, 1280 mg, or 1500 mg, more preferably 600 mg or 750 mg.

The dosing frequency of the PD-L1 antibody or antigen-binding fragment thereof is once every 2 weeks or one every 3 weeks.

In some embodiments, the fixed dose of the PD-L1 antibody or antigen-binding fragment thereof is 490-2000 mg, and the dosing frequency is once every 2 weeks or once every 3 weeks; preferably, the dose is 490mg, 500mg, 550mg, 600mg, 750mg, 1200mg, 1280mg or 1500mg, and the dosing frequency is once every 2 weeks; or the dose is 490 mg, 500 mg, 550 mg, 600 mg, 750 mg, 1200 mg, 1280 mg or 1500 mg, and administrated once every 3 weeks; more preferably, the dose is 600 mg or 750 mg, and the dosing frequency is once every 2 weeks or once every 3 weeks.

In some embodiments, the administration allows the target patient population to achieve the following average pharmacokinetic (pk) distribution:
For the pharmacokinetic (pk) distribution of the PD-L1 antibody or antigen-binding fragment thereof, the average Cₘₐₓ is about 269.75 µg/mL (±20%), and/or the median time to reach a maximum concentration (Tₘₐₓ) is about 0.54 hours (±20%) (such as 0.08 to about 2 hours), and/or the area under the mean plasma concentration-time curve (AUC ₀₋₂₁) is about 1985.61 µg.day/mL (±20%) from the time of dosing (time 0) to about 21 hours after dosing, and/or the area under the mean plasma concentration-time curve (AUC_{inf} ) is about 3568.48 µg.day/mL (±20%) from the time of dosing (time 0) to infinite time after dosing.

The present disclosure also relates to a pharmaceutical composition comprising the IL-15 or protein complex thereof, the PD-L1 antibody or antigen-binding fragment thereof, and one or more pharmaceutical carrier(s), excipient(s) or diluent(s). The pharmaceutical composition can be formulated as any pharmaceutically acceptable dosage form. For example, it can be formulated as tablet, capsule, pill, granule, solution, suspension, syrup, injection agent (including injection solution, sterile powder for injection and concentrated solution for injection), suppository, inhalant or spray agent.

The pharmaceutical composition comprising an IL-15 or protein complex thereof, and a PD-L1 antibody or antigen-binding fragment thereof described in the present disclosure can be administrated alone or in combination with one or more therapeutic agents.

The present disclosure also provides a kit in which the IL-15 or protein complex thereof and the PD-L1 antibody or antigen-binding fragment thereof of the present disclosure are packaged.

In the present disclosure, an IL-15 or protein complex thereof administrated in combination with a PD-L1 antibody or antigen-binding fragment thereof thereby enhances the anti-tumor activity and improves the therapeutic effects on tumor diseases.

### Detailed description

Three-letter codes and one-letter codes for amino acids used in the present disclosure are as described in J. biol. chem, 243, p3558 (1968).

As used herein, "antibody" refers to immunoglobulin, a four-peptide chain structure which is formed by two identical heavy chains and two identical light chains connected together by interchain disulfide bond(s). Immunoglobulin heavy chain constant regions exhibit different amino acid compositions and orders, hence present different antigenicity. Accordingly, immunoglobulins can be divided into five types, or named as immunoglobulin isotypes, namely IgM, IgD, IgG, IgA and IgE, and the corresponding heavy chains are µ, δ, γ, α and ε chain, respectively. According to the amino acid composition of hinge region as well as the number and location of heavy chain disulfide bonds, the same type of Ig can further be divided into different sub-types, for example, IgG can be divided into IgG1, IgG2, IgG3 and IgG4. Light chains can be divided into κ or λ chain based on different constant regions. Each of the five types of Ig has a kappa chain or a lambda chain.

In the present disclosure, the antibody light chain described in the present disclosure may further include a light chain constant region, and the light chain constant region includes human or murine kappa, lambda chain or variant thereof.

In the present disclosure, the antibody heavy chain described in the present disclosure may further include a heavy chain constant region, and the heavy chain constant region includes human or murine IgG1, IgG2, IgG3, IgG4 or variant thereof.

About 110 amino acid adjacent to the N-terminus of the antibody heavy and light chains are highly variable, known as variable regions (Fv regions); the rest of amino acid sequences close to the C-terminus are relatively stable, known as constant regions. The variable region includes three hypervariable regions (HVRs) and four relatively conserved framework regions (FRs). The three hypervariable regions which determine the specificity of the antibody are also known as complementarity determining regions (CDRs). Each of the light chain variable region (LCVR) and heavy chain variable region (HCVR) consists of 3 CDR regions and 4 FR regions, with the sequential order from the amino terminus to carboxyl terminus of: FR1, CDR1, FR2, CDR2, FR3, CDR3, and FR4. The three CDR regions of the light chain refer to LCDR1, LCDR2, and LCDR3, and the three CDR regions of the heavy chain refer to HCDR1, HCDR2, and HCDR3. The number and position of the CDR amino acid residues of the LCVR region and the HCVR region of the antibody or antigen-binding fragment thereof described in the present disclosure comply with the known Kabat numbering criteria.

The antibodies of the present disclosure include murine antibodies, chimeric antibodies, and humanized antibodies, preferably humanized antibodies.

The term "humanized antibody", also known as CDR-grafted antibody, refers to an antibody generated by grafting the murine CDR sequences onto human antibody variable region frameworks, i.e., an antibody produced in different types of human germline antibody framework sequences. It avoids strong responses to antibody variable regions induced by chimeric antibody which carries a large number of murine protein components. Such framework sequences can be obtained from public DNA database covering germline antibody gene sequences or published references. For example, germline DNA sequences of human heavy and light chain variable region genes can be found in "VBase" human germline sequence database (available on www.mrccpe.com.ac.uk/vbase), as well as in Kabat, EA, et al. 1991 Sequences of Proteins of Immunological Interest, 5th Ed. To avoid a decrease in activity caused by the decreased immunogenicity, the framework sequences in human antibody variable region may be subjected to minimal reverse mutations or back mutations to maintain the activity. The humanized antibody of the present disclosure also comprises humanized antibody on which CDR affinity maturation is performed by phage display.

The "antigen-binding fragment thereof" as described in the present disclosure refers to Fab fragments, Fab' fragments, F(ab')2 fragments, and Fv fragments that bind to human PD-L1 and ScFv fragments that have antigen-binding activity; it comprises one or more CDR regions selected from SEQ ID NO:1 to SEQ ID NO:12 of the antibodies described in the present disclosure. The Fv fragment, which contains the antibody heavy and light chain variable region, without constant regions, is the minimal antibody fragment comprising all the antigen-binding sites. Generally, Fv antibodies also contain a polypeptide linker between the VH and VL domains, and can form the structure required for antigen binding. Different linkers can also be used to connect the variable regions of two antibodies to form a polypeptide chain, which is referred to as single chain antibody or single chain Fv (sFv). The term "binding to PD-L1" as used in the present disclosure refers to the ability to interact with human PD-L1. The term "antigen-binding site" described in the present disclosure refers to a discrete three-dimensional spatial site present on an antigen that is recognized by the antibody or antigen-binding fragment thereof of the present disclosure.

The "protein complex" or "complex protein" as used in the present disclosure refers to a protein formed by binding two different monomeric proteins. The "monomer protein" (i.e. soluble fusion protein (I) and the soluble fusion protein (II)) which forms the protein complex of present disclosure can be a fusion protein or a non-fusion protein.

The "fusion protein" as described in the present disclosure refers to a protein product obtained by connecting the coding regions of two or more genes and expressing the recombinant gene under the control of an identical regulatory sequence via gene recombination methods, chemical methods or other appropriate methods. In some embodiments of the present disclosure, the soluble fusion protein (I) is a monomeric protein obtained by expressing an IL-15 or variant thereof fused or not fused to a biologically active polypeptide, such as an Fc fragment; and the soluble fusion protein (II) is a monomeric protein obtained by expressing an IL-15Rα or variant thereof fused or not fused to a biologically active polypeptide, such as an Fc fragment. Within the fusion protein of the present disclosure, the coding regions of the two or more genes can be fused to each other at one or more position(s), via a sequence encoding a peptide linker. Peptide linkers can also be used to construct the fusion proteins of the present disclosure.

"Administration" or "treatment," as it applies to an animal, human, experimental subject, cell, tissue, organ, or biological fluid, refers to contacting an exogenous pharmaceutical, therapeutic, diagnostic agent, or composition with the animal, human, subject, cell, tissue, organ, or biological fluid. "Administration" and "treatment" can refer, e.g., to therapeutic, pharmacokinetic, diagnostic, research, and experimental methods. The treatment of a cell involves contacting a reagent with the cell, as well as contacting a reagent with a fluid, and the fluid in turn is in contact with the cell. "Administration" or "treatment" also means *in vitro* or *ex vivo* treatments, e.g., of a cell, with a reagent, diagnostic, binding composition, or with another cell. "Treatment", as it applies to a human, veterinary, or research subject, refers to therapeutic treatment, prophylactic or preventative measures, research and diagnostic applications.

"To treat" means to apply a therapeutic agent (such as a composition comprising any of binding compounds of the present disclosure), internally or externally, to a patient having one or more disease symptoms for which the agent has known therapeutic activity. Typically, the agent is administrated in an amount effectively to alleviate one or more disease symptoms in the patient or population to be treated, to induce the regression of or inhibit the progression of such symptom(s) by any clinically measurable degree. The amount of a therapeutic agent that is effective to alleviate any particular disease symptom (also referred to as the "therapeutically effective amount") may vary according to various factors such as the disease state, age, and body weight of the patient, and the ability of the agengt to elicit a desired response in the patient. Whether a disease symptom has been alleviated can be assessed by any clinical measurement typically used by physicians or other skilled healthcare providers to assess the severity or progression status of that symptom. While an embodiment of the present disclosure (e.g., a treatment method or article of manufacture) may not be effective in alleviating the target disease symptom(s) in every patient, it should alleviate the target disease symptom(s) in a statistically significant number of patients as determined by any statistical test known in the art such as Student's t-test, chi-square test, U-test according to Mann and Whitney, Kruskal-Wallis test (H-test), Jonckheere-Terpstra-test and Wilcoxon-test.

The "combination" described in the present disclosure means a mode of administration, which refers to the administration of at least one dose of the IL-15 or protein complex thereof and at least one dose of the PD-L1 antibody or antigen-binding fragment thereof within a certain period of time, wherein both agents show pharmacological effects. Said period of time may be within one dosing period, preferably within 4 weeks, within 3 weeks, within 2 weeks, within 1 week, or within 24 hours, more preferably within 12 hours. The IL-15 or protein complex thereof and the PD-L1 antibody or antigen-binding fragment thereof can be administrated simultaneously or sequentially. Within such period of time, treatments are involved, in which the IL-15 or protein complex thereof and the PD-L1 antibody or antigen-binding fragment thereof are administrated via the same or different administration routes. The combined administration mode of the present disclosure is selected from the group consisting of simultaneous administration, co-administration of separate formulations, and sequential administration of separate formulations.

"Effective amount" encompasses an amount sufficient to ameliorate or prevent a symptom or sign of the medical condition. Effective amount also means an amount sufficient to allow or facilitate diagnosis. An effective amount for a particular patient or veterinary subject may vary depending on factors such as the condition being treated, the overall health condition of the patient, the route and dose of administration and the severity of side effects. An effective amount can be the maximal dose or dosing protocol that avoids significant side effects or toxic effects.

### DESCRIPTION OF THE DRAWINGS

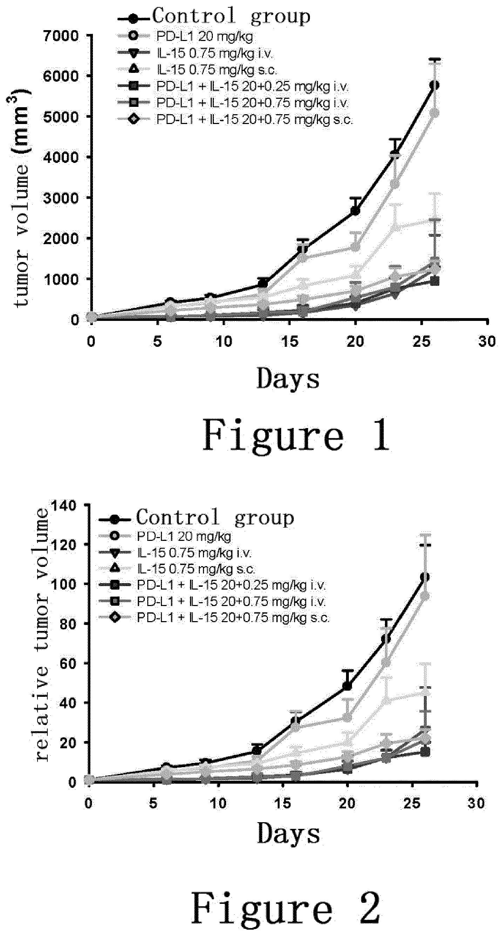

Figure 1. The effect of the IL-15 fusion protein and the PD-L1 antibody administrated alone or in combination on tumor volume in a tumor model subcutaneously transplanted with mouse colon cancer cell MC38/H11 in Example 1.
Figure 2. The effect of the IL-15 fusion protein and the PD-L1 antibody administrated alone or in combination on the relative tumor volume in a tumor model subcutaneously transplanted with mouse colon cancer cell MC38/H11 in Example 1.
Figure 3. The C1D15 receptor occupancy of the PD-L1 antibody in Example 3;
Figure 4. The C1D21 receptor occupancy of the PD-L1 antibody in Example 3;

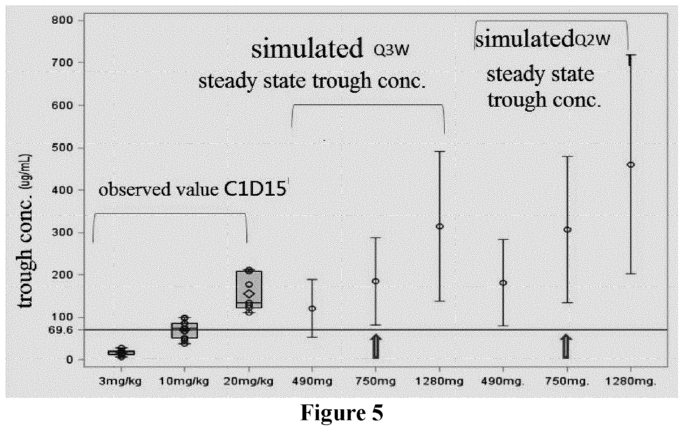

Figure 5. A comparison of the observed and simulated values with the minimum effective concentration of the PD-L1 antibody, in Example 3.

### DETAILED DESCRIPTION OF THE INVENTION

### Example 1: Study concerning the therapeutic effect of the IL-15 protein complex and the anti-PD-Ll antibody (administrated in combination or administrated alone) on transplanted tumor of mouse colon cancer cell MC38/H11 in mice

### 1. Test agent

IL-15 fusion protein: the sequence of which is as shown in No. 3 and consists of soluble fusion protein (I) (SEQ ID NO: 14) and soluble fusion protein (II) (SEQ ID NO: 17). Preparation method: 1.0 mL of water for injection was injected into lyophilized powder of IL-15 fusion protein (1.0 mg) to prepare a 1.0 mg/mL solution by fully dissolving. 1.5 mL of 1.0 mg/mL IL-15 fusion protein solution was added with 13.5 mL of saline and fully mixed to obtain 20 mL of 0.075 mg/mL solution. 0.25 mL of 1.0 mg/mL IL-15 fusion protein solution was added with 9.75 mL saline, and mixed well to obtain 10 mL of 0.025 mg/mL solution.

PD-L1 antibody: the heavy chain sequence of which is SEQ ID NO: 9, and the light chain sequence of which is SEQ ID NO: 11. Preparation method: 0.8 mL of 50 mg/mL PD-L1 antibody solution was added with 19.2 mL of 5% glucose solution, and mixed well to obtain 20 mL of 2 mg/mL solution.

hIgG1: 0.075 mg/mL solution was prepared with saline.
hIgG4-Fc: 2 mg/mL solution was prepared with 5% glucose solution.

### 2. Laboratory animals

C57BL/6 mice, 8-10 weeks old, female, were purchased from Zhejiang Charles River Laboratory Animal Technology Co., Ltd. Laboratory animal certificate number: 1806280013. Feeding environment: SPF level; temperature: the temperature was controlled at 20 to 26°C; relative humidity: the relative humidity was controlled at 40% to 70%; light: automatic lighting, with 12/12 hours of light/dark cycle.

### 3. Experimental procedures

1×10⁶ MC38/H11 mouse colon cancer cells (mouse endogenous PD-L1 was knocked out from mouse colon cancer cell line MC38 by using CRISPR/Cas9 technology, and then human PD-L1 gene was transferred to make MC38 cells stably express human PD-L1. H-11 is a constructed monoclonal cell line after screening) were injected into the left armpit of the mouse. When the tumor grew to an average volume of 50-100 mm³ , 63 mice were divided into 7 groups: negative control group (Control), PD-L1 antibody 20 mg/kg group, IL-15 fusion protein 0.75 mg/kg (i.v.) group, IL-15 fusion protein 0.75 mg/kg (s.c.) group, PD-L1 antibody + IL-15 fusion protein 20 + 0.25 mg/kg (i.v.) group, PD-L1 antibody + IL-15 fusion protein 20 + 0.75 mg/kg (i.v.) group and PD-L1 antibody + IL-15 fusion protein 20+0.75 mg/kg (s.c.) group, with 9 animals each group. The corresponding concentration of the test substance was administrated to each group with an administration volume of 10 mL/kg. Body weight and the tumor volume were measured twice a week, and the dosing period was 25 days. On day 26, the body weight and the tumor volume were measured, and the relative tumor volume (RTV) and the relative tumor growth rate (T/C) were calculated and subjected to statistical analysis. The calculation formula is as follows:
(1) TV (tumor volume) =1/2×a×b², where a and b represent the length and width of the tumor respectively;
(2) RTV (relative tumor volume) =Vₜ/V₀ , where Vo refers to the tumor volume measured on the grouping day (i.e. do), and Vt refers to the tumor volume measured each time;
(3) T/C (%) =T_{RTV} /C_{RTV} × 100%, where T_{RTV} refers to the RTV of the treatment group, and C_{RTV} refers to the RTV of the control group;
(4) TGI (Tumor Growth Inhibition) (%) = (1- T_{RTV} /C_{RTV})×100%, where T_{RTV} refers to the RTV of the treatment group, and C_{RTV} refers to the RTV of the control group.

### 4. Experimental results

| **Group** | **dose (mg/kg)** | **Tumor volume (mm³)** | | **RTV** | **T/C (%)TGI(%)** | |
|---|---|---|---|---|---|---|
| | | **D1** | **D26** | | | |
| Control group (hIgG1+hIgG4-Fc) | 20+0.75 | 60±4 | 5758±647 | 103.28±16.25 | - | - |
| PD-L1 antibody | 20 | 60±5 | 5073±1223 | 93.65±31.08 | 90.67 | 9.33 |
| IL-15 Fusion Protein | 0.75 (i.v.) | 59±5 | 1238±840 *** | 26.53±21.09 ** | 25.69 | 74.31 |
| | 0.75 (s.c.) | 59±6 | 2445±652 ** | 45.11+14.50 * | 43.68 | 56.32 |
| PD-L1 antibody + IL-15 fusion protein (i.v.) | 20+0.25 | 58±5 | 948±345 ***^{###} | 15.20±4.87 **^{##} | 14.72 | 85.28 |
| | 20+0.75 | 58±5 | 1425±1032 ***^{##} | 21.23±14.39 **^{##} | 20.55 | 79.45 |
| PD-L1 antibody + IL-15 fusion protein (s.c.) | 20+0.75 | 58±6 | 1233:1:211***^{###□△} | 22.27±4.26**^{##□△} | 21.56 | 78.44 |

Compared to the control group, : *P* <0.05, : *P* <0.01, : *P* <0.001 (One-way ANOVA); compared to the PD-L1 antibody 20 mg/kg group, ^{##} : *P* <0.01, ^{###} : *P* <0.001 (One-way ANOVA); Compared to the IL-15 fusion protein 0.75 mg/kg (i.v.) group , PD-L1 antibody + IL-15 fusion protein 20+0.75 mg/kg (i.v.) group shows no statistical difference (T-test); compared to the IL-15 fusion protein 0.75 mg/kg (s.c.) group, ^{□} : *P* <0.05 (T-test); compared to the PD-L1 antibody + IL-15 fusion protein 20+0.75 mg/kg (i.v.) group, ^{△}: *P* <0.05 (T-test).

Experimental results show that the IL-15 fusion protein 0.25 mg/kg (i.v.), 0.75 mg/kg (i.v. and s.c.) in combination with the PD-L1 antibody can significantly improve the inhibitory effect of the PD-L1 antibody on the growth of subcutaneously transplanted tumor of MC38/H11 mouse colon cancer cells in mice.

### Example 2: Phase I clinical study of tolerance, safety, pharmacokinetics and pharmacodynamics of the IL-15 fusion protein in combination with the PD-L1 antibody in patients with advanced malignant tumors

### 1. Test agents

IL-15 fusion protein: the sequence of which is as shown in No. 3 and consists of soluble fusion protein (I) (SEQ ID NO: 14) and soluble fusion protein (II) (SEQ ID NO: 17). Specification: 1mg/vial.

The PD-L1 antibody has a heavy chain sequence of SEQ ID NO: 9 and a light chain sequence of SEQ ID NO: 11. Specification: 600mg/vial.

### 2. Enrolled subjects

(1) 18 to 70 years old, male and female;
(2) Patients who have been diagnosed with advanced/metastatic malignant tumors by histopathology or cytology;
(3) Patients who experienced failed standard treatment or was intolerant to the standard treatment, and there was no more standard treatment once disease relapse/progression.

### 3. Method of administration

The qualified subjects after the screening were administrated with the IL-15 fusion protein and the PD-L1 antibody.

The IL-15 fusion protein was administrated at 1µg/kg, 3µg/kg, 6µg/kg or 10µg/kg by intravenous infusion, or at 3µg/kg, 6µg/kg, 10µg/kg, 15µg/kg or 20µg/kg by subcutaneous injection, once a week; the PD-L1 antibody was administrated by intravenous infusion at 600 mg, once every two weeks. During the administration, the IL-15 fusion protein was firstly administrated as a single-agent loading for 2 weeks or 4 weeks, and then the IL-15 fusion protein was administrated in combination with the PD-L1 antibody.

### Example 3: Phase I clinical study of tolerance, safety, pharmacokinetics and pharmacodynamics of the PD-L1 antibody in patients with advanced solid tumors

### 1. Test agents

The PD-L1 antibody has a heavy chain sequence of SEQ ID NO: 9 and a light chain sequence of SEQ ID NO: 11. Specification: 600mg/vial.

### 2. Enrolled subjects

(1) Over 18 years old;
(2) Patients who have been diagnosed with metastatic or locally advanced solid tumors by histology or cytology;
(3) Patients who experienced failed standard treatment, or patients for whom the existing anti-tumor treatment was ineffective.

### 3. Method of administration

The qualified subjects after the screening were administrated with the PD-L1 antibody, and every three weeks were considered as a dosing period. On day 1 in each dosing period, the PD-L1 antibody was administrated via intravenous infusion within 30 minutes, at a dose of 1 mg/kg, 3 mg/kg, 10 mg/kg or 20 mg/kg.

### 4. Experimental results

Dose-limiting toxicity (DLT) was not observed at any dose level. The disease control rate was 40.0%, and the objective response rate was 8.6%. The pharmacokinetic data are shown in the table below.

| Parameter | The dose of the PD-L1 antibody | | | |
|---|---|---|---|---|
| | 1 mg/kg | 3 mg/kg | 10 mg/kg | 20 mg/kg |
| Number of subjects (n) | 1 | 12 | 12 | 12 |
| AUC_{inf} (µg.day/mL) | 205.75 | 731.57±332.8 | 3568.48±1761.96 | 7532.96±2565.61 |
| AUC₀₋₂₁ (µg.day / mL) | 182.82 | 490.31±155.66 | 1985.61±477.46 | 3791.57±1049.05 |
| Cmax (µg/mL) | 27.8 | 69.83±15.78 | 269.75±58.38 | 489.92±100.86 |
| Tmax (hr) | 1.0 | 1 (0.08-1) | 0.54 (0.08-2) | 1 (0.08-2) |
| Vd (mL/kg) | 44.43 | 63.92±15.97 | 62.87±13.82 | 70.74±23.9 |
| CL (mL/kg/day) | 4.86 | 5.36±3.47 | 3.52±1.82 | 3.04±1.34 |
| T (day) | 6.94 | 11.28±5.73 | 17.08±12.07 | 19.74±12.06 |
| MRT (day) | 9.14 | 15.5±8.11 | 23.34±16.56 | 27.47±16.53 |

Body weight of the test patients:

| Parameter | Value |
|---|---|
| Number of subjects | 37 |
| Average weight (kg) | 74.30 |
| Median weight (kg) | 73.78 |
| Std. (kg) | 16.95 |
| CV (%) | 22.82 |

The therapeutic effect and side effect of each dose group are as follows. The therapeutic effect and side effect of the PD-L1 antibody are not significantly correlated with the increase of dose.

| **Dose** | **Number of subjects** | **SD** | **PR** | **PD** | **Not evaluated** |
|---|---|---|---|---|---|
| 3mg/kg | 12 | 5 (41%) | 1 (8.3%) | 6 (50%) | 0 |
| 10mg/kg | 12 | 6 (50%) | 0 | 4 (33.3%) | 2 (16.7%) |
| 20mg/kg | 12 | 5 (41%) | 0 | 2 (16.7%) | 5 (41%) |

| **Adverse events** | **Number of adverse events** | | |
|---|---|---|---|
| | **3 mg/kg (n=12)** | **10 mg/kg (n=12)** | **20 mg/kg (n=12)** |
| **All adverse events** | **77** | **61** | **32** |
| Adverse events irrelative to the administration | 64 (83.1%) | 48 (78.7%) | 24 (75%) |
| Adverse events related to the administration | 13 (16.9%) | 13 (21.3%) | 8 (25%) |
| Severity level 1 | 53 (68.8%) | 44 (72.1%) | 23 (71.9%) |
| Severity level 2 | 15 (19.5%) | 15 (24.6%) | 6 (18.8%) |
| Severity level 3 | 7(9.1%) | 1 (1.6%) | 2 (6.3%) |
| Severity level 4 | 0 | 0 | 0 |
| Severity level 5 (death) | 2 (2.6%) | 1 (1.6%) | 1 (3.1%) |

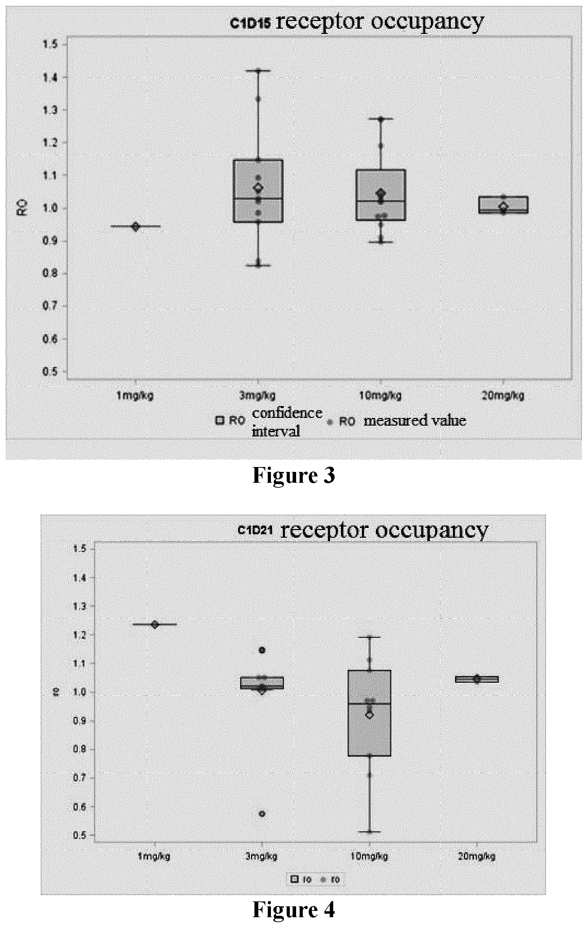

The whole blood samples were collected from the patients after administration, and the PD-L1 Receptor occupancy (RO) on the surface of peripheral blood T cells was detected after the administration of PD-L1 antibody by using flow cytometry (Figure 3), and the minimum effective concentration of the PD-L1 antibody was confirmed as about 70 µg/mL. The patient's blood-drug concentration was fitted with two-compartment model, and a series of trough concentrations were obtained for various doses in the fixed-dose administration method, as shown in the following table.

| | Observed value | | | simulated Q3W | | | simulated Q2W | | |
|---|---|---|---|---|---|---|---|---|---|
| | 3mg/kg | 10mg/kg | 20mg/kg | 490mg | 750mg | 1280mg | 490mg | 750mg | 1280mg |
| Average blood-drug concentration (µg/mL) | 16 | 70 | 156 | 121 | 185 | 315 | 181 | 307 | 460 |
| Maximum blood-drug concentration/90% upper CI | 28 | 100 | 212 | 188 | 288 | 492 | 283 | 480 | 718 |
| Minimum blood-drug concentration/90% lower CI | 6 | 36 | 111 | 53 | 81 | 138 | 79 | 135 | 202 |

The blood-drug concentrations of the PD-L1 antibody were compared to the minimum effective concentration. In all dosing regimen, with the exception of the Q3W 490mg regimen, the minimum blood-drug concentration were all higher than the minimum effective concentration of PD-L1 antibody, indicating that all the dosing regimens can meet the needs of clinical treatment. Therefore, the PD-L1 antibody can be administrated in a fixed-dose mode.

## Claims

1. Use of an IL-15 or protein complex thereof in combination with a PD-L1 antibody or antigen-binding fragment thereof in the preparation of medicament for the prevention or treatment of tumor diseases.

2. The use according to claim 1, wherein any one of the PD-L1 antibody or antigen-binding fragment thereof comprises CDRs selected from the following CDR region sequences or mutant sequences thereof: antibody heavy chain variable region HCDR region sequences: SEQ ID NOs: 1-3; and antibody light chain variable region LCDR region sequences: SEQ ID NOs: 4-6; in particular,
| | |
|---|---|
| HCDR1 is selected from: SYWMH | SEQ ID NO: 1 |
| HCDR2 is selected from: RI X₁PNSG X₂TSYNEKFKN | SEQ ID NO: 2 |
| HCDR3 is selected from: GGSSYDYFDY | SEQ ID NO: 3 |
| LCDR1 is selected from: RASESVSIHGTHLMH | SEQ ID NO: 4 |
| LCDR2 is selected from: AASNLES | SEQ ID NO: 5 |
| LCDR3 is selected from: QQSFEDPLT | SEQ ID NO: 6; |
wherein X₁ is selected from H or G, preferably G; X ₂ is selected from G or F, preferably F.

3. The use according to claim 2, wherein the heavy chain variable region sequence of the PD-L1 antibody or antigen-binding fragment thereof is SEQ ID NO: 7, and the light chain variable region sequence is SEQ ID NO :8.

4. The use according to claim 3, wherein the PD-L1 antibody or antigen-binding fragment thereof further comprises a heavy chain constant region of human IgG1, IgG2, IgG3 or IgG4 or a variant thereof, preferably comprises a heavy chain constant region of human IgG2 or IgG4, more preferably comprises a heavy chain constant region of IgG4 having F234A and L235A mutations; and the humanized antibody light chain further comprises a constant region of human kappa, lambda chain or a variant thereof.

5. The use according to claim 4, wherein the heavy chain sequence for the PD-L1 antibody or antigen-binding fragment thereof is SEQ ID NO: 9, and the light chain sequence is SEQ ID NO :11.

6. The use according to claim 1, wherein the PD-L1 antibody or antigen-binding fragment thereof is selected from the group consisting of a murine antibody, a chimeric antibody, a humanized antibody and a human antibody, preferably a humanized antibody.

7. The use according to claim 1, wherein the IL-15 protein complex is composed of a soluble fusion protein (I) and a soluble fusion protein (II); wherein the soluble fusion protein (I) comprises an IL-15 polypeptide or functional fragment thereof; the soluble fusion protein (II) comprises an IL-15Rα polypeptide or functional fragment thereof; one or more amino acid site(s) of the soluble fusion protein (I) or the soluble fusion protein (II) is/are mutated into Cys, which forms disulfide bond(s) with Cys at the corresponding amino acid site(s) of the soluble fusion protein (II) or soluble fusion protein (I).

8. The use according to claim 7, wherein the soluble fusion protein (II) further comprises an Fc fragment or a mutant thereof; preferably, the soluble fusion protein (II) is composed of an IL-15Rα polypeptide or functional fragment thereof connected to the N-terminus of the Fc fragment; more preferably, the Fc fragment is shown as SEQ ID NO: 13.

9. The use according to claim 7, wherein the sequence of the soluble fusion protein (I) is SEQ ID NO:14.

10. The use according to any one of claims 7 to 9, wherein the sequence of the soluble fusion protein (II) is selected from the group consisting of SEQ ID NO: 15, SEQ ID NO: 16, SEQ ID NO: 17 and SEQ ID NO: 18.

11. The use according to claim 7, wherein the IL-15 protein complex is selected from the following combinations of the soluble fusion protein (I) and the soluble fusion protein (II):
| Numbering | Soluble fusion protein (I) | Soluble fusion protein (II) |
|---|---|---|
| 1 | IL-15(L52C)(SEQ ID NO: 14) | IL-15Rα-ECD(S40C)-Fc(SEQ ID NO: 15) |
| 2 | IL-15(L52C)(SEQ ID NO: 14) | Fc-IL-15Rα-ECD(S40C)(SEQ ID NO: 16) |
| 3 | IL-15(L52C)(SEQ ID NO: 14) | IL-15Rα-Sushi+(S40C)-Fc(SEQ ID NO:17) |
| 4 | IL-15(L52C)(SEQ ID NO: 14) | Fc-IL-15Rα-sushi+(S40C)(SEQ ID NO:18). |

12. The use according to claim 8, the IL-15 protein complex is selected from the combination of IL-15 (L52C) (SEQ ID NO: 14) soluble fusion protein (I) and IL-15Rα-Sushi+ (S40C)-Fc (SEQ ID NO: 17) soluble fusion protein (II).

13. The use according to any one of claims 1-12, wherein the tumor disease is selected from the group consisting of malignant tumor and benign tumor, preferably selected from the group consisting of melanoma, skin cancer, renal cell carcinoma, liver cancer, gastric cancer, breast cancer, colorectal cancer, glioblastoma, ovarian cancer, prostate cancer, hematologic malignancy, urothelial/bladder cancer, lung cancer, esophageal cancer, and head and neck cancer.

14. The use according to any one of claims 1-13, wherein the tumor is selected from the group consisting of advanced tumors, relapsed and refractory tumors, tumors that experienced failed treatment of chemotherapy agent and/or relapsed, tumors that experienced failed radiotherapy and/or relapsed, tumors that experienced failed therapy of targeted agent and/or relapsed, and tumors that experienced failed immunotherapy and/or relapsed.

15. The use according to any one of claims 1-14, wherein the tumor is selected from advanced or metastatic malignancy.

16. The use according to any one of claims 1-15, wherein the dose of the IL-15 or the protein complex thereof is selected from 1µg/kg to 100 µg/kg, preferably 1µg/kg, 3µg/kg, 6µg /kg, 10µg/kg, 15µg/kg or 20µg/kg.

17. The use according to any one of claims 1-16, wherein the dose of the PD-L1 antibody or antigen-binding fragment thereof is selected from 50 mg to 3000 mg, preferably 600 mg or 750 mg.

18. A pharmaceutical composition comprising the IL-15 or the protein complex thereof, the PD-L1 antibody or antigen-binding fragment thereof of claim 1, and one or more pharmaceutically acceptable excipient(s), diluent(s) or carrier(s).
